(19) Europäisches Patentamt
European Patent Office
Office européen des brevets

(11) Publication number: 0 426 290 A2

(12) EUROPEAN PATENT APPLICATION

(21) Application number: 90310155.8

(22) Date of filing: 17.09.90

(51) Int. Cl.[5]: C07C 49/04, C07C 31/125, C07C 45/28, C07C 29/48

(30) Priority: 30.10.89 US 428701
30.10.89 US 428812
30.10.89 US 428703

(43) Date of publication of application:
08.05.91 Bulletin 91/19

(84) Designated Contracting States:
DE FR GB IT

(71) Applicant: TEXACO CHEMICAL COMPANY
3040 Post Oak Boulevard
Houston, Texas 77056(US)

(72) Inventor: Sanderson, John Ronald
P.O. Box 587
Leander, Texas 78641(US)
Inventor: Marquis, Edward Thomas
9004 Collinfield Drive
Austin, Texas 78758(US)
Inventor: Paynton, Howard Franklin
4409 Walhill Lane
Austin, Texas 78759(US)
Inventor: Knifton, John Frederick
10900 Catskill Trail
Austin, Texas 78726(US)

(74) Representative: Brock, Peter William et al
URQUHART-DYKES & LORD 91 Wimpole Street
London W1M 8AH(GB)

# (54) Process for the production of detergent range alcohols and ketones.

(57) Detergent range alcohols and ketones are obtained by reacting an alkane having 10 to 18 carbon atoms with a hydroperoxide in the presence of catalysts which are transition metal compounds selected from:

(a) dicyano bis-(1,10)-phenanthrolene iron (II),

(b) iron, ruthenium or chromium acetylacetonate, and

(c) an iron, manganese or cobalt phthalocyanine or metallo-porphine.

EP 0 426 290 A2

# PROCESS FOR THE PRODUCTION OF DETERGENT RANGE ALCOHOLS AND KETONES

The invention relates to the catalytic production of detergent range alcohols and ketones, and more particularly the production of detergent range alcohols and ketones from alkanes and hydroperoxides in the presence of certain transition metal catalysts.

Producing alchols and/or ketones from alkanes and other co-reactants is known. Often, such technology is pursued in attempts to discover viable processes for making useful chemicals directly from hydrocarbons, in the expectation of dwindling petrochemical feedstocks. In other instances, the co-reactant may be a by-product from another process and an economical way of converting the by-product into a useful material may be needed. Sometimes a particular kind of alcohol or ketone, or mixture of alcohols and/or ketones, is desired.

A number of publications provide a useful background in this technology. For example, A.N. Bashikirov, et al in "Synthesis of Higher Aliphatic Alcohols bv Direct Oxidation of Paraffinic Hydrocarbons", Proc. World Pet. Cong., Vol. 4 (1959) pp. 175-183, report the "directed" synthesis of higher aliphatic alcohols via the liquid-phase oxidation of paraffinic hydrocarbons in the presence of boric acid, and by the selection of proper operating conditions, which include a low oxygen concentration. Apparently, the boric acid serves as an esterification agent in the oxidation, and the conversion of alcohols into boric acid esters prevents them from further oxidation by interrupting the oxidative conversion chain at the alcohol stage. See also N.J. Stevens, et al ., "A New Route for Alcohols", Chemical Engineering Progress, Vol. 64, No. 7, (1968) p. 61-66. Similarly, US-A-3243449 teaches the oxidation of saturated hydrocarbons having 4 to 8 carbon atoms, with molecular oxygen in the presence of metaboric acid or a less hydrated form of orthoboric acid, including boric anhydride, to produce borate esters. In this process, the contact temperature for the reactants is from 140 to 180 °C, and the reaction is controlled to restrict tne partial pressure of water in the exit gases, i.e., the vapor above the liquid reaction mixture.

In this same area is work reported by M.J. Ijam, et al ., in "Liquid-Phase Oxidation of n-Dodecane in the Presence of Boron Compounds", Ind. Eng. Chem. Prod. Res. Dev., Vol. 20, (1981) pp. 315-319. The paper describes experiments on the production of neutral oxidation products rich in alcohols by the direct air oxidation of n-dodecane in the presence of boron compounds, such as tributoxyboroxine, boron trioxide, or dibutoxyborane. As with some of the previously discussed publications, lean oxygen-nitrogen mixtures (4% oxygen) are also used, here giving a mixture of six possible straight-chain $C_{12}$ alcohols.

In the absence of boric acid or similar catalysts, the direct oxidation of hydrocarbons produces alcohols and ketones in usually less than 60% selectivity, even at conversions of 20 to 30%. In the presence of boric acid, the selectivity to alcohols is increased to 75 to 80% at 20 to 30% conversion, but even here, carboxylic acids and other by-products are produced. In addition, the hydrolysis of the borate esters and isolation of the products is not simple.

Paraffins having 4 to 8 carbon atoms may be oxidized in the liquid phase with molecular oxygen in a reactor in which the manganese content is maintained in the range of 2 to 50 ppm to produce carboxylic acids, according to US-A-3859346. The manganese may be in the form of carboxylic acid salts, e.g. manganese naphthenate, or in an aqueous solution, e.g. manganese acetate.

Also of general interest is "Porphyrin Catalysts for Olefin Epoxidation: A literature review 1985-86", Catalysts in Chemistry, Vol. 21, No. 3, (1987) pp. 106-112. The oxidation processes briefly mentioned include ones invol ving iron and manganese porphyrin catalysts, primarily with a concentration on processes producing epoxides. The oxidation of cyclohexane to cyclohexanol and cyclohexanone by molecular oxygen, catalyzed by ruthenium (III)-ethylene-diaminetetraacetic acid in the presence and absence of the micelle cetyltrimethylammonium bromide (CTAB), is reported by M.M. Taqui Kahn, et al. in "Ru(III)-EDTA Catalyzed Oxidation of Cyclohexane by Molecular Oxygen", Journal of Molecular Catalysis, Vol. 45, (1988) pp. 51-56. The rate of oxidation was found to increase in the presence of CTAB. Similarly T. Lau et al. in "Ruthenium Catalysed Oxidation of Alkanes with Alkylhydroperoxides", J. Chem. Soc., Chem. Commun., (1988) pp. 1406-1407, report that cis-$[Ru(II)(L)_2(OH_2)_2]^{2+}$ complexes may catalyze the oxidation of saturated hydrocarbons, such as cyclohexane, hexane and heptane, to alcohols and ketones by t-butylhydroperoxide. The L in the ruthenium catalyst formula may be substituted 2,2′-bipyridines or 1,10-phenanthrolines.

Of somewhat more pertinent interest are a number of publications describing research focussing on metalloporphyrin catalysts to make alcohols and ketones. For example, D. Mansuy, et al . in "Metalloporphyrin-Catalyzed Hydroxylation of Cyclohexane by Alkyl Hydroperoxides: Pronounced Efficiency of Iron-Porphyrins", Angew. Chem. Int. Ed. Engl., Vol. 19 No. 11, (1980) pp. 909-910, describe the catalyzed hydroxylation of nonactivated alkanes, either by molecular oxygen in the presence of a reducing

agent, or by two-electron oxidants. The researchers noted that the metalloporphyrins studied fell into three classes. First, the Cu(II)-, Ni(II)-, Zn(II)-, Mg(II)-, V(IV)- and Ti(IV)-porphyrins were found to be completely inactive under the reaction conditions used. The Co(TPP) and Os(TPP)(CO)(PY) compounds were found to catalyze cyclohexane oxidation, the former giving even a slightly faster reaction and better yields than Fe-(TPP)(Cl), but both showed decreasing activity over time. The third group, Fe- and Mn(TPP)Cl, were found to be true catalysts. A related study is reported in D. Mansuy, et al., "Alkane Hydroxylation Catalyzed by Metalloporphyrins: Evidence for Different Active Oxygen Species with Alkylhydroperoxides and Iodosobenzene as Oxidants", Tetrahedron Letters, Vol. 23, No. 27 (1982) pp. 2781-2784. The comparative examination of cyclohexane and n-heptane hydroxylations by cumylhydroperoxide and iodosobenzene, catalyzed by various metalloporphyrins, indicated that different active oxygen species, presumably the cumyloxy radical and a metal-oxo intermediate, were involved in these reactions. The metals used in the catalysts included iron, manganese, cobalt, rhodium and chromium.

Azido(tetraphenylporphyrinato) complexes of $Cr^{III}$, $Mn^{III}$ and $Fe^{III}$ are found to catalyze the selective, low temperature hydroxylation of isobutane with molecular oxygen, according to P.E. Ellis, et al. in "Effect of Axial Azide on the Selective, Low Temperature Metalloporphyrin-Catalysed Reactions of Isobutane with Molecular Oxygen", J. Chem. Soc., Chem. Commun., 1989, pp. 1187-88.

P.H.J. Carlsen in "Ruthenium Catalyzed Oxidation of Alkanes", Synthetic Communications, Vol . 17, No. 1, (1987) pp. 19-23 reports the use of a $RuCl_3$ catalyst in a solvent system containing $CCl_4$-$CH_3CN$-$H_2O$, and using sodium metaperiodate as the stoichiometric oxidation agent, to oxidize a series of alkyl-substituted alkanes. Norbornane and bicyclo[2.2.2]octane are oxidized to the corresponding ketones, and adamantane is transformed into 1-adamantol. Another article of interest is Y.V. Geletti, et al., "Oxidation of Saturated Hydrocarbons by Hydrogen Peroxide in Pyridine Solution Catalysed by Copper and Iron Perchlorates", J. Chem. Soc., Chem. Comm., (1988) pp. 936-937. The hydrocarbons used are cyclohexane and 2-methylbutane, which yield the ketone and alcohol, with alkyl radicals not being intermediates.

Additionally of note is US-A-4459427 which teaches that a mixture of alcohols and ketones may be produced by reacting a $C_2$ to $C_{10}$ alkane with a hydrocarbyl hydroperoxide, e.g. t-butyl hydroperoxide, in the presence of a catalyst. The catalyst may be an iron or manganese square planar complex having heterocyclic nitrogen-donor ligands, e.g. a porphyrin or phthalocyanine complex, which complex has either no axial ligands, for example, the lower valency or cationic complex, or an axial ligand which is non-coordinating or weakly-coordinating. Weakly-coordinating ligands are defined as ligands having a coordinating power less than that of the chloride anion. The alkane employed is preferably a linear or branched alkane having from 2 to 20 carbon atoms. Suitable complexes having no axial ligands are either neutral iron (II) and manganese (II) complexes, e.g. Fe(II)(TPP) and Mn(II)(TPP), or iron (III) and manganese (III) cationic complexes, such as $[Fe(III)(TPP)]^+$ and $[Mn(III)(TPP)]^+$.

US-A-3879467 describes a process for the catalytic oxidation of alkanes, using a hydroperoxide and a chromium catalyst, especially chromium acetylacetonate. The claims of US-A-3879467 are restricted to the oxidation of certain low molecular weight alkanes and cyclohexane.

EP-A-0274909 indicates that hydrocarbons, particularly lower molecular weight alkanes and cycloalkanes, may be oxidized with air or oxygen to form products such as alcohols ketones and the like selectively in high yields when the catalyst is a highly active azide- or nitride-activated metal coordination complex having the structure:

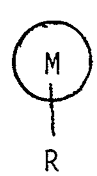

where M is a transition metal; the circle represents a ligand and R is azide or nitride. Certain dimeric forms of these catalysts may also be employed. It is also mentioned that Group IV to VIII transition metal nitrides are effective oxidation catalysts for lower molecular weight hydrocarbons, such as alkanes. The discussion therein is also directed to certain novel azide-activated metal coordination complex catalysts, per se.

It will be appreciated that in the processes briefly described above, a wide variety of products is often obtained. In many of the reports on those processes, it was stated that a mixture of all possible products resulted. Thus, it would be advantageous if processes could be developed which would maximize the yield to a particularly useful product, in turn, by minimizing the yield to the other by-products. It would also be preferred that the reaction be able to be run at ambient temperature with catalysts that are readily

obtainable so that the synthesis might be relatively simple and economical.

This invention relates to a process for the production of a mixture of detergent range alcohols and ketones by reacting an alkane having 10 to 18 carbon atoms with a hydroperoxide in the presence of a catalyst. The catalyst is a transition metal compound selected from:

(a) dicyano bis-(1,10)-phenanthrolene iron (II),

(b) iron, ruthenium or chromium acetylacetonate, and

(c) an iron, manganese or cobalt phthalocyanine or metalloporphine, e.g. ferrous phthalocyanine, chloroferric phthalocyanine, iron (II) mesotetraphenyl porphine chloride, manganese (II) tetraphenyl porphine acetate, cobalt (II) mesotetraphenyl porphine or a mixture thereof.

It has been discovered that detergent range alcohols and ketones may be produced, from hydrocarbons and a hydroperoxide with few by-products, when using the above-mentioned catalysts. In general, alkanes are difficult to react, and consequently in many of the oxidation techniques discussed previously and those employed herein, a preparation step is required to provide an appropriate feedstock or co-reactant. In a preparation step, a second hydrocarbon containing a tertiary carbon, different from that used to make the alcohols and ketones, e.g. cumene or tertiary-butane, is oxidized to the corresponding hydroperoxide. Next, the hydroperoxide is reacted with the hydrocarbon in the presence of the catalyst, in accordance with the present invention. After isolation of the alcohols and ketones by distillation, the by-product carbinol derived from the hydroperoxide is reduced to the hydrocarbon and recycled to the hydroperoxide formation step. The overall result is the production of alcohols and ketones with a minimum of by-product formation. For example, dodecanones and dedecanols from n-dodecane. The alcohols and ketones produced are useful intermediates for surfactants and detergents.

The second step, the reaction of interest, may be outlined as follows:

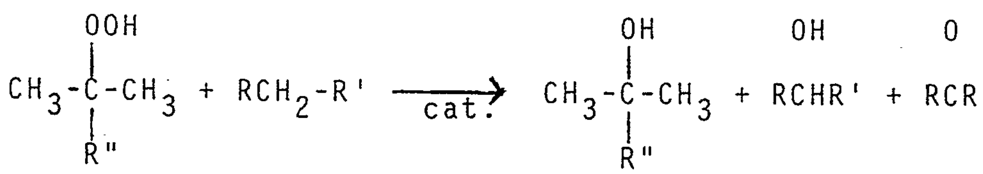


where R and R′ are alkyl groups, which taken together and with the central $CH_2$ group have a total of 10 to 18 carbon atoms, and where R″ is an alkyl or aryl group of 1 to 10 carbon atoms. $RCH_2R'$ may be a linear or branched hydrocarbon. In one embodiment, these hydrocarbons have 11 to 16 carbon atoms.

Examples of suitable hydroperoxides are cumene hydroperoxide, t-butyl hydroperoxide and mixtures thereof; and examples of suitable alkanes are decane, undecane, n-dodecane, tridecane and tetradecane, although it will be appreciated that the inventive process is not limited to these illustrative examples. As mentioned, the hydroperoxides may be prepared by the conventional air oxidation of the corresponding hydrocarbon. In one aspect, good results are obtained when the hydroperoxide is concentrated at least in the range of 60 to 90%. With respect to the hydrocarbons, are hydrocarbons or mixtures may be employed.

In one embodiment, the catalyst used according to the invention is dicyano bis-(1,10)-phenanthrolene iron(II). The reaction conditions advantageous for this embodiment are a temperature of 10 to 180° C, preferably 20 to 100° C or even 20 to 80° C. Atmospheric pressures are preferred for convenience, but elevated pressures may be suitable as well, with certain modifications to the process.

In another embodiment, certain transition metal acetylacetonate catalysts have been discovered as being suitable for the reaction. Ruthenium acetylacetonate $Ru(AcAc)_3$ , ferric or iron acetylacetonate Fe-$(AcAc)_3$ , and chromium acetylacetonate $Or(AcAc)_3$ can be used. Certain ligands, co-catalysts and other additives, when used in conjunction with these catalyst systems, may provide beneficial results. Further, copper acetate $Cu(OAc)_2$ has been found to be a useful co-catalyst together with iron acetylacetonate.

In this embodiment also, the advantageous reaction conditions are a temperature from 10 to 180° C, preferably 20 to 100° C or even 20 to 80° C, while atmospheric pressures are preferred for convenience, but elevated pressures can be employed.

In another embodiment of the invention, iron, manganese or cobalt phthalocyanines and metalloporphines are employed. Specific examples of suitable catalysts include, but are not necessarily limited to, iron phthalocyanine, ferrous phthalocyanine, chloroferric phthalocyanine, iron (II) meso-tetraphenyl porphine chloride, manganese (III) tetraphenyl porphine acetate, cobalt (II) meso-tetraphenyl porphine, and mixtures thereof. Interestingly, nickel phthalocyanine does not give the desired alcohol and/or ketone product. It has also been discovered that certain ligands, oxidants and other additives, when used in conjunction with these catalyst systems, may provide beneficial results. For example, it has been discovered that the metalloporphines mentioned above work particularly well with imidazoles as ligands. Further, chloroferric phthalocyanine may use lithium borate as a ligand, or may use potassium superoxide or iodosylbenzene as an additive to advantage. Alkali metal perchlorates, such as sodium perchlorate have also been discovered as useful additives when employed with chloroferric phthalocyanine. When potassium superoxide, $KO_2$, is employed together with the chloroferric phthalocyanine catalyst, tetrabutyl ammonium bromide may advantageously be added to improve the yield of the ketone/alcohol mixture. Other suitable superoxides that may be of use include, but are not limited to, rubidium superoxide and cesium superoxide. Although the superoxides and iodosyl benzene are both known as oxidants, it is not believed that they act as oxidants in this case. One possible way in which these additives might function is as "reactive ligands" in that they may perform as the borates or imidazoles, i.e. as ligands, but also be reactive to some extent.

The reaction conditions advantageous for this embodiment are a temperature from 10 to 180 °C, preferably 20 to 80 °C. Atmospheric pressures are preferred for convenience, but elevated pressures may be suitable. It has been found, with some of the catgalysts, such as chloroferric phthalocyanine and ferrous phthalocyanine, that at higher temperatures, e.g. at 80 °C or above, more alcohol than ketone is produced, whereas at lower temperatures, e.g. at 20 °C or below, the ratio shifts to favour the ketone. This discovery is contrary to expectation, since one skilled in the art would expect that more ketone, the more highly oxidized species, would be formed at the higher temperatures.

As noted previously, an advantage of the process is that the carbinol may be converted back into the corresponding hydrocarbon, and recycled to the oxidation step to produce more hydroperoxide. To take cumene hydroperoxide as a specific example, the by-product dimethyl phenyl carbinol may be reduced to cumene, using known technology, and recycled.

The invention is useful for the preparation of higher molecular weight alcohols and ketones, which are useful chemical intermediates for surfactants and detergents. Additionally, there is a minimum of by-product derived from the hydrocarbon. Various esters of the alcohols are useful lubricants, and some serve as plasticizers for plastics. The reaction takes place at a temperature in the range from about 50 °C to about 110 °C, with 80 °C being a preferred temperature. The invention will be described further with reference to the following illustrative, detailed examples.

## PROCEDURE 1A

This procedure will describe the preparation of the hydroperoxide, using cumene hydroperoxide (CHP) as an example. Cumene (190.0g 99% pure provided by Aldrich Chemical Co.) and cumene hydroperoxide (10.0g 85.7% pure provided by Aldrich Chemical Co.) were charged to a 250 ml resin flask equipped with stirrer, thermometer, heating mantle, water cooled condenser, and gas-sparging tube. The reaction mixture was purged with nitrogen and heated to the desired temperature (±2 °C). A zero-time sample with withdrawn for gas chromatography (GC) analysis, and air (or pure oxygen) was sparged through the reaction mixture at the required rate, using a Gilmont flowmeter. The reaction mixture was heated at the desired temperature (±2 °C) and small samples (0.2 - 0.5 g) were withdrawn at various times. The samples were flushed with nitrogen and placed in small sealed vials until analysis by GC. At the end of the reaction, the reaction mixture was cooled to ambient temperature (while nitrogen was flushed through the solution) and then placed in a tared bottle. GC analysis was on a Hewlett-Packard 5890 gas chromatograph. The column was a 0.53 mm x 30 meter fused silica capillary column (DB-17, 50% phenyl methyl silicone). The conditions: 80-275 °C after 4.0 min. 10 # He 190 cc split, 0.5 µl injection, 80 °C injection temperature. Some typical results are shown in Table 1A. The examples are numbered starting at Example 101 to easily distinguish them from the more important inventive examples, which begin at "Example 1". Cumene may be distilled off to produce CHP of the desired concentration for use in the Examples 1 to 90 according to the invention.

TABLE 1A

Peroxidation of Cumene

| Ex. No. | Time (hrs) | Temp. °C | Fl Rt[1] (ml/m) | Oxygen Form | Cumene Conv.% | ----Selectivity %[2]---- CHP | DMPC | ACETP | MeSTY | UNK |
|---|---|---|---|---|---|---|---|---|---|---|
| 101 | 1.00 | 110.0 | 100.0 | A | 1.63 | 90.31 | 6.85 | 1.77 | 0.53 | 0.00 |
| 102 | 2.00 | 110.0 | 100.0 | A | 4.47 | 94.00 | 3.99 | 1.09 | 0.34 | 0.06 |
| 103 | 3.00 | 110.0 | 100.0 | A | 7.90 | 95.13 | 3.17 | 0.86 | 0.25 | 0.07 |
| 104 | 4.00 | 110.0 | 100.0 | A | 11.82 | 94.63 | 3.68 | 0.92 | 0.21 | 0.06 |
| 105 | 5.00 | 110.0 | 100.0 | A | 15.78 | 93.91 | 4.12 | 1.23 | 0.18 | 0.06 |
| 106 | 6.00 | 110.0 | 100.0 | A | 20.52 | 92.69 | 4.65 | 1.90 | 0.15 | 0.10 |
| 107 | 7.00 | 110.0 | 100.0 | A | 24.19 | 92.19 | 5.21 | 1.44 | 0.14 | 0.10 |
| 108 | 0.50 | 120.0 | 100.0 | A | 1.76 | 92.79 | 4.57 | 1.54 | 0.50 | 0.00 |
| 109 | 1.00 | 120.0 | 100.0 | A | 3.57 | 92.97 | 4.37 | 1.62 | 0.46 | 0.07 |
| 110 | 2.00 | 120.0 | 100.0 | A | 7.63 | 92.78 | 4.56 | 1.64 | 0.41 | 0.09 |
| 111 | 3.00 | 120.0 | 100.0 | A | 12.54 | 92.73 | 4.77 | 1.54 | 0.35 | 0.11 |
| 112 | 4.00 | 120.0 | 100.0 | A | 16.57 | 91.66 | 5.55 | 1.77 | 0.33 | 0.17 |
| 113 | 5.00 | 120.0 | 100.0 | A | 20.67 | 90.55 | 6.45 | 2.03 | 0.29 | 0.17 |
| 114 | 6.00 | 120.0 | 100.0 | A | 24.98 | 88.78 | 7.21 | 2.35 | 0.26 | 0.32 |
| 115 | 7.00 | 120.0 | 100.0 | A | 28.88 | 85.07 | 6.38 | 6.80 | 0.24 | 0.35 |
| 116 | 0.50 | 130.0 | 100.0 | A | 1.32 | 90.70 | 5.30 | 2.09 | 1.22 | 0.00 |
| 117 | 1.00 | 130.0 | 100.0 | A | 3.18 | 91.44 | 4.82 | 1.98 | 1.02 | 0.16 |
| 118 | 1.50 | 130.0 | 100.0 | A | 5.33 | 90.69 | 5.44 | 2.28 | 0.90 | 0.17 |
| 119 | 2.00 | 130.0 | 100.0 | A | 7.89 | 90.16 | 5.90 | 2.47 | 0.79 | 0.18 |
| 120 | 3.00 | 130.0 | 100.0 | A | 12.43 | 86.91 | 7.48 | 3.47 | 0.70 | 0.22 |
| 121 | 4.00 | 130.0 | 100.0 | A | 17.53 | 85.07 | 9.63 | 3.91 | 0.59 | 0.29 |
| 122 | 5.00 | 130.0 | 100.0 | A | 23.14 | 81.44 | 11.53 | 4.61 | 0.47 | 0.50 |
| 123 | 6.00 | 130.0 | 100.0 | A | 28.31 | 78.99 | 13.97 | 5.21 | 0.38 | 0.64 |
| 124 | 1.00 | 140.0 | 100.0 | A | 4.56 | 87.48 | 7.28 | 3.34 | 1.38 | 0.00 |
| 125 | 2.00 | 140.0 | 100.0 | A | 9.48 | 80.96 | 12.15 | 5.10 | 1.26 | 0.00 |

[1] CHP = cumene hydroperoxide, DMPC = dimethylphenylcarbinol, ACETP = acetophenone, MeSTY = α-methylstyrene, UNK = sum of unknowns with retention higher than CHP.
[2] Flow rate for air (A) or pure oxygen (B).

| Ex. No. | Time (hrs) | Temp. °C | Fl Rt[1] (ml/m) | Oxygen Form | Cumene Conv.% | Selectivity %[2] CHP | DMPC | ACETP | MeSTY | UNK |
|---|---|---|---|---|---|---|---|---|---|---|
| 126 | 1.00 | 110.0 | 200.0 | B | 1.41 | 96.65 | 2.24 | 0.56 | 0.00 | 0.00 |
| 127 | 2.00 | 110.0 | 200.0 | B | 4.71 | 95.12 | 3.21 | 0.86 | 0.21 | 0.05 |
| 128 | 3.00 | 110.0 | 200.0 | B | 9.16 | 94.47 | 3.88 | 0.94 | 0.16 | 0.04 |
| 129 | 4.00 | 110.0 | 200.0 | B | 13.62 | 93.82 | 4.45 | 1.04 | 0.14 | 0.05 |
| 130 | 5.00 | 110.0 | 200.0 | B | 18.74 | 93.17 | 5.08 | 1.09 | 0.12 | 0.04 |
| 131 | 6.00 | 110.0 | 200.0 | B | 23.19 | 92.44 | 5.42 | 1.48 | 0.11 | 0.05 |
| 132 | 1.00 | 120.0 | 200.0 | B | 6.22 | 93.72 | 4.05 | 1.35 | 0.28 | 0.06 |
| 133 | 2.00 | 120.0 | 200.0 | B | 15.67 | 91.15 | 5.47 | 1.74 | 0.18 | 0.07 |
| 134 | 3.00 | 120.0 | 200.0 | B | 26.74 | 89.99 | 7.19 | 2.13 | 0.13 | 0.07 |
| 135 | 4.00 | 120.0 | 200.0 | B | 37.51 | 87.09 | 8.84 | 2.46 | 0.10 | 0.09 |
| 136 | 5.00 | 120.0 | 200.0 | B | 46.41 | 85.83 | 13.19 | 0.00 | 0.12 | 0.16 |
| 137 | 6.00 | 120.0 | 200.0 | B | 53.16 | 82.16 | 15.76 | 0.00 | 0.20 | 0.40 |
| 138 | 1.00 | 130.0 | 200.0 | B | 14.51 | 90.57 | 6.12 | 2.46 | 0.24 | 0.08 |
| 139 | 2.00 | 130.0 | 200.0 | B | 36.36 | 85.27 | 9.06 | 4.90 | 0.12 | 0.14 |
| 140 | 3.00 | 130.0 | 200.0 | B | 55.21 | 79.77 | 14.61 | 4.38 | 0.12 | 0.26 |
| 141 | 4.00 | 130.0 | 200.0 | B | 64.87 | 67.65 | 12.48 | 15.21 | 0.49 | 2.21 |
| 142 | 5.00 | 130.0 | 200.0 | B | 65.57 | 50.07 | 22.72 | 9.94 | 1.40 | 10.86 |
| 143 | 1.00 | 140.0 | 200.0 | B | 25.92 | 86.01 | 8.99 | 4.03 | 0.26 | 0.21 |
| 144 | 2.00 | 140.0 | 200.0 | B | 53.19 | 75.31 | 11.98 | 11.41 | 0.16 | 0.54 |
| 145 | 3.00 | 140.0 | 200.0 | B | 64.33 | 59.88 | 27.95 | 7.67 | 0.55 | 1.93 |

PROCEDURE 1B

A mixture of tert-butyl hydroperoxide, tert-butyl alcohol and acetone in a ratio of 65:34:1, equivalent to 5-6% isobutane conversion, and finely divided sodium pyrophosphate (0.01 wt.% basis total) was charged to the autoclave through a small vent hole near the top of the reactor. The autoclave was then sealed and isobutane pressured in. The mixture was then heated to the desired temperature. The stirring rate was 300 rpm. Oxygen was added in ~1 g increments until a pressure of 1.0 to 1.4 MPa (150-200 psi) over autogeneous was reached. Oxygen was then added only after the pressure had dropped by 0.34 MPa (50 psi). The reaction was continued for the desired time and near the end of the reaction, no more oxygen was added so that a large excess of oxygen would not be present to create an explosive mixture. The reaction was cooled as rapidly as possible to ambient temperature and the contents pressured out into a tared stainless steel bomb with nitrogen at 2.17 MPa (300 psi ). The weight % products were determined by GC analysis of bomb contents.

TABLE 1B

| Isobutane Oxidation at 145 °C | | | | | |
|---|---|---|---|---|---|
| | | | ----------Products----------- | | |
| Ex. | Time (Hr) | IB (Wt.%) | THBP (Wt.%) | TBA (Wt.%) | Acetone[1] (wt.%) |
| 146 | 2.0 | 73.915 | 18.114 | 7.678 | 0.155 |
| 147 | 2.5 | 69.745 | 20.847 | 9.012 | 0.197 |
| 148 | 3.5 | 59.101 | 26.964 | 13.188 | 0.406 |
| 149 | 4.0 | 55.917 | 28.347 | 14.972 | 0.460 |
| 150 | 2.0 | 74.712 | 17.751 | 7.188 | 0.135 |
| 151 | 2.5 | 70.923 | 19.815 | 8.833 | 0.285 |
| 152 | 3.0 | 65.161 | 23.227 | 10.858 | 0.271 |
| 153 | 4.0 | 55.307 | 28.742 | 15.247 | 0.427 |
| 154 | 4.0 | 55.207 | 28.494 | 15.526 | 0.448 |
| 155 | 2.0 | 75.814 | 17.130 | 6.850 | 0.122 |
| 156 | 3.0 | 66.495 | 22.604 | 10.453 | 0.251 |
| 157 | 4.0 | 55.593 | 28.188 | 13.720 | 0.422 |
| 158 | 4.0 | 53.980 | 28.728 | 15.613 | 0.499 |
| 159 | 1.0 | 83.183 | 12.252 | 4.468 | 0.037 |
| 160 | 3.0 | 63.706 | 23.693 | 11.716 | 0.309 |
| 161 | 3.16 | 62.065 | 24.478 | 12.352 | 0.332 |

[1]Acetone plus methanol, but methanol content is usually low (20-15% of acetone).

## EXAMPLES 1 TO 10

Detergent range alcohols and ketones were produced as follows. A 250 ml flask was charged with the indicated catalyst(s), n-dodecane (30.0 g), and tert-butyl alcohol (35.0 g), and this mixture was vigorously stirred. A mixture of 33.0 g of 90% tert-butyl hydroperoxide and 22.0 g of tert-butyl alcohol was added slowly to this stirred mixture over several hours. The reactions at 25 °C were controlled by means of a water bath. The reactions at 80 °C were controlled by means of a heating mantle. The reaction mixtures were stirred for a total of 20 hours and filtered through fluted filter paper. The results shown in Table 2 were obtained by GPC.

TABLE 2

| Examples 1 to 10 | | | | | | | | | |
|---|---|---|---|---|---|---|---|---|---|
| | | | | | ---------Products, Wt.% by GPC--------- | | | | |
| Ex. | Catalyst | Cat. (g) | Time (hr) | Temp °C | TBHP | Ketone | Alcohol | Kt&Al Total | DTBP |
| 1 | Fe(II)DCPA | 0.20 | 20 | 25 | 17.57 | ≈0 | ≈0 | ≈0 | ≈0 |
| 2 | Fe(II)DCPA | 0.50 | 20 | 25 | 18.09 | ≈0 | ≈0 | ≈0 | ≈0 |
| 3 | Fe(II)DCPA | 0.20 | 20 | 80 | 1.93 | 3.51 | 1.76 | 5.27 | 0.64 |
| 4 | Fe(II)DCPA | 0.50 | 20 | 80 | 0.92 | 3.25 | 1.37 | 5.62 | 0.56 |
| 5 | Fe(II)DCPA | 0.05 | 20 | 80 | 4.07 | 3.41 | 1.12 | 4.26 | 0.48 |
| 6 | Fe(II)DCPA | 0.10 | 20 | 80 | 1.10 | 3.25 | 1.74 | 4.99 | 0.53 |
| 7 | $Fe(AcAc)_3$ | 0.50 | 20 | 80 | 14.74 | 0.50 | 0.36 | 0.86 | 0.11 |
| 8 | Fe2[COL.]3[CIT.]3 | 0.20 | 20 | 80 | 17.16 | 0.26 | 0.28 | 0.54 | ≈0 |
| 9 | Fe2[COL.]3[CIT.]3 | 0.50 | 20 | 80 | 16.98 | 0.11 | ≈0 | ≈0.11 | ≈0 |
| 10 | Fe(III)DETAPA | 0.50 | 20 | 80 | 17.17 | 0.14 | 0.04 | 0.18 | 0.07 |

It is of interest that although Fe(II)DCPA gave good yields of alcohols and ketones in Examples 1 to 6, other iron compounds, such as iron acetylacetonate - $Fe(AcAc)_3$, iron choline citrate - $Fe_2[COL.]_3[CIT.]_3$, and diethylenetriamine pentaacetic acid iron(III) disodium salt -Fe(III)DETAPA, did not give good yields in the other examples.

## EXAMPLES 11 TO 15

n-dodecane (50.0 g) and the indicated catalyst(s) were charged to a 250 ml flask equipped with stirrer, dropping funnel and thermometer. Cumene hydroperoxide (80%) was added slowly to the stirred reaction mixture over several hours. The temperature was maintained by the use of a water bath or a heating mantle. At the end of the reaction, the mixture was filtered and analyzed by GC and/or GC/IR (infra-red spectroscopy). The results are shown in the following Table 3. The alcohols and ketones may be isolated by distillation. The by-products dimethyl phenyl carbinol and α-methyl styrene may be reduced and recycled.

TABLE 3

| | | | | | Wt.% $C_{12}$ Products | |
|---|---|---|---|---|---|---|
| Ex. | Catalyst(s)[1] | Wt., grams | Time hours | Temp °C[2] | Ketone | Alcohol |
| 11 | $Ru(AcAc)_3$ | 0.05 | 20.0 | 30 | 2.68 | 0.79 |
| 12 | $Ru(AcAc)_3$ | 0.05 | 23.0 | 30 | 1.19 | 0.45 |
| | IM | 0.025 | | | | |
| 13 | $Ru(AcAc)_3$ | 0.05 | 23.0 | 30 | 0.41 | 0.12 |
| | IM | 0.05 | | | | |
| 14 | $Ru(AcAc)_3$ | 0.05 | 23.0 | 30 | 2.45 | 0.48 |
| | $Fe(AcAc)_3$ | 0.02 | | | | |
| 15 | $Ru(AcAc)_3$ | 0.05 | 23.0 | 30 | 2.43 | 0.40 |
| | $Cr(AcAc)_3$ | 0.02 | | | | |

[1] $Ru(AcAc)_3$ = ruthenium acetylacetonate
$Cr(AcAc)_3$ = chromium acetylacetonate
$Fe(AcAc)_3$ = ferric acetylacetonate
IM = imidazole
[2] ±5 °C

Interestingly, the yields using imidazole as a ligand with ruthenium acetylacetonate, in Examples 12 and 13, were poorer than when ruthenium acetylacetonate was used alone (Example 11).

## EXAMPLES 16 TO 19

These next Examples will illustrate the use of copper acetate and iron acetylacetonate each alone and together as co-catalysts.

Procedure: a 250 ml flask was charged with catalyst(s), n-dodecane (30.0 g), tert-butyl alcohol (35.0 g), and this mixture was vigorously stirred. Next, 55.0 g of 54.6% tert-butyl hydroperoxide solution in tert-butyl alcohol was added over several hours. The reactions at 25 °C were controlled by a water bath. The reactions at 80 °C were controlled by means of a heating mantle. The reactors were stirred for a total of 20 hours. At the end of the reaction, the reaction mixture was analyzed by GPC. The results are shown in Table 4.

TABLE 4

| Ex | Catalyst | (g) | Temp °C | Wt.% TBHP | Wt.% Ketone | Wt.% Alcohol | Wt.% Total | Wt.% DTBP |
|---|---|---|---|---|---|---|---|---|
| 16 | $Cu(OAc)_2$ | 0.50 | 25 | 17.77 | 0.094 | 0 | 0.094 | 0.12 |
| 17 | $Fe(AcAc)_3$ | 0.50 | 80 | 14.74 | 0.50 | 0.036 | 0.86 | 0.11 |
| 18 | $Fe(AcAc)_3$ | 0.50 | 80 | 11.51 | 1.44 | 0.98 | 2.42 | 0.26 |
| | $Cu(OAc)_2$ | 0.10 | | | | | | |
| 19 | $Fe(AcAc)_3$ | 0.50 | 80 | 7.16 | 2.75 | 1.45 | 4.20 | 0.40 |
| | $Cu(OAc)_2$ | 0.05 | | | | | | |

It is of interest that a greater yield to ketone and alcohol products is achieved with a combination of the copper acetate and iron acetylacetonate catalysts than with either catalyst alone.

## EXAMPLES 20 TO 30

n-dodecane (50.0 g) and the indicated catalyst(s) were charged to a 250 ml flask equipped with stirrer, dropping funnel and thermometer. Cumene hydroperoxide (80%) was added slowly to the stirred reaction mixture over several hours. The temperature was maintained by the use of a water bath or a heating mantle. At the end of the reaction, the mixture was filtered and analyzed by GC and/or GC/IR (infrared spectroscopy). The results are shown in the following Table 5. The alcohols and ketones may be isolated by distillation. The by-products dimethyl phenyl carbinol and α-methyl styrene may be reduced and recycled.

TABLE 5

| | | | | | Wt.% $C_{12}$ Products | |
|---|---|---|---|---|---|---|
| Ex. | Catalyst(s)[1] | Wt. grams | Time hours | Temp °C | Ketone | Alcohol |
| 20 | FePCYCl<br>IM | 0.10<br>0.07 | 24.0 | 30 | 5.02 | 1.42 |
| 21 | FePCYCl<br>IM | 0.10<br>0.025 | 23.0 | 30 | 4.39 | 0.98 |
| 22 | Fe(II)PCY<br>IM | 0.10<br>0.05 | 23.0 | 30 | 4.57 | 0.90 |
| 23 | FePCYCl | 0.10 | 7.0 | 60 | 3.04 | 1.04 |
| 24 | FePCYCl<br>IM | 0.10<br>0.05 | 7.0 | 60 | 2.61 | 1.24 |
| 25 | Ni(II)PCY<br>IM | 0.10<br>0.05 | 24.0 | 30 | 0 | 0 |
| 26 | $Fe(AcAc)_3$ | 0.10 | 26.0 | 26 | 0.161 | 0 |
| 27 | FePCYCl | 0.10 | 24.0 | 26 | 4.40 | 1.00 |
| 28 | Fe(III)TPPCl<br>IM | 0.05<br>0.025 | 25.0 | 30 | 1.75 | 0.24 |
| 29 | Mn(III)TPPOAc | 0.05 | 23.0 | 30 | 1.47 | 0.328 |
| 30 | Co(III)TPP<br>IM | 0.05<br>0.025 | 26.0 | 30 | 1.18 | 0.31 |

[1]FePCYCl = chloroferric phthalocyanine
Fe(II)PCY = ferrous phthalocyanine
Ni(II)PCY = nickel (II) phthalocyanine
$Fe(AcAc)_3$ = ferric acetyl acetonate
Fe(III)TPPCl = iron (III) m-tetraphenyl porphine
Mn(III)TPPOAc = manganese (III) m-teraphenyl porphine acetate
Co(III)TPP = cobalt (II) m-tetraphenyl porphine
IM = imidazole

Interestingly, the nickel (II) phthalocyanine catalyst used in Example 25 gave neither the desired alcohol nor the desired ketone, although the iron porphyrins did give the alcohol and ketone products; see Examples 20 to 24 and 27 to 30. Also by way of contrast, the iron acetylacetonate catalyst used in Example 26 gave no alcohol and very little ketone.

## EXAMPLES 31 TO 39

A 250 ml flask was charged with the indicated catalyst(s), n-dodecane (30.0 g), tert-butyl alcohol (35.0 g), and this mixture was vigorously stirred. A mixture of 33.0 g of 90% tert-butyl hydroperoxide and 22.0 g of tert-butyl alcohol were added slowly to this stirred mixture over several hours. All of the reactions were performed at 25°C and were controlled by means of a water bath. The reaction mixtures were stirred for a

total of 20 hours and filtered through fluted filter paper. The results shown in Table 6 were obtained by GPC. Chloroferric phthalocyanine [Fe(III)PCYCl] in quantities of 0.50 g was used in all of these Examples. It is believed that the lithium borate serves as a ligand in complex with the chloroferric phthalocyanine.

TABLE 6

| Ex. | $LiBO_2$ grams | Wt.% TBHP | Wt.% Ketone | Wt.% Alcohol | Wt.% Total | Wt.% DTBP |
|---|---|---|---|---|---|---|
| 31 | None | 0.05 | 4.26 | 0.92 | 5.18 | 1.96 |
| 32 | 0.1 | 0.06 | 6.11 | 1.10 | 7.21 | 1.51 |
| 33 | 0.1 | 0.04 | 6.68 | 0.99 | 7.67 | 1.82 |
| 34 | 0.20 | 0 | 6.56 | 1.11 | 7.67 | 1.60 |
| 35 | 0.25 | 0 | 7.40 | 1.37 | 8.77 | 1.23 |
| 36 | 0.50 | 0 | 6.21 | 1.02 | 7.23 | 1.66 |
| 37 | 0.50 | 0 | 6.70 | 1.25 | 7.95 | 1.58 |
| 38 | 1.00 | 0 | 7.14 | 1.33 | 8.47 | 1.47 |
| 39 | None | 0.13 | 4.34 | 0.70 | 5.04 | 2.03 |

It will be appreciated that the use of lithium borate in Examples 32 to 38 gives noticeably higher yields of the desired ketones and alcohols than are obtained when chloroferric phthalocyanaine is employed alone, as in Examples 31 and 39. It is also noted that, when the lithium borate was employed in quantities of at least 0.2 g under these conditions, essentially all of the tert-butyl hydroperoxide (TBHP) is reacted, as seen in Examples 34 to 38.

## EXAMPLES 1T TO 25T

These examples illustrate the discovery that the ratio of ketone/alcohol product may be adjusted by changing the reaction temperature. Two reaction temperatures are used: 20 °C and 80 °C. At the higher temperature, more alcohol is formed, and at the lower temperature, more ketone is formed. These experiments were all conducted according to the following procedure.

A 250 ml flask equipped with stirrer, thermometer, condenser, and dropping funnel was charged with n-dodecane (30.0 g), t-butyl alcohol (50.0 g), and the indicated catalyst(s). t-Butyl hydroperoxide (74.3%, 40.0 g) was then added over 4 to 5 hours. The mixture was then stirred overnight. The temperature control was within ±5 °C. The reaction mixture was then filtered and analyzed by GC. The results are shown in Table 7. The Examples are designated with the notation "T" to indicate that they illustrate the temperature effect of this invention.

TABLE 7

| Ex. | Catalyst I | (g) | Cat. II | (g) | Time (hr) | Temp (°C) | Wt.% TBHP | Wt.% Ket. | Wt.% Alc. | Wt.% Total | Wt.% Ket/Alc. | Wt.% DTBP |
|---|---|---|---|---|---|---|---|---|---|---|---|---|
| 1T | FePCYCl | 0.25 | - | - | 20 | 25 | - | 5.75 | 1.16 | 6.91 | 4.96 | 1.80 |
| 2T | FePCYCl | 0.50 | CuPCY | 0.10 | 20 | 25 | ~0 | 7.53 | 1.54 | 9.07 | 4.89 | 1.18 |
| 3T | CuPCY | 0.50 | - | - | 20 | 25 | 17.77 | 0.094 | 0 | 0.094 | - | 0.12 |
| 4T | FePCYCl | 0.50 | - | - | 20 | 80 | ~0 | 3.27 | 5.27 | 8.54 | 0.62 | 0.16 |
| 5T | FePCYCl | 0.50 | CuPCY | 0.05 | 20 | 80 | ~0 | 3.98 | 5.94 | 9.92 | 0.67 | 0.19 |
| 6T | FePCYCl | 0.50 | CuPCY | 0.05 | 20 | 25 | ~0 | 8.16 | 1.77 | 9.93 | 4.61 | 1.19 |
| 7T | FePCYCl | 0.50 | CuPCY | 0.10 | 20 | 25 | ~0 | 7.19 | 1.26 | 8.45 | 5.70 | 1.33 |
| 8T | $Fe(AcAc)_3$ | 0.50 | - | - | 20 | 80 | 14.74 | 0.50 | 0.36 | 0.86 | - | 0.11 |
| 9T | $Fe(AcAc)_3$ | 0.50 | $Cu(AcAc)_2$ | 0.10 | 20 | 80 | 11.51 | 1.44 | 0.98 | 2.42 | - | 0.26 |
| 10T | FePCYCl | 0.25 | - | - | 20 | 25 | ~0 | 6.20 | 1.19 | 7.39 | 5.21 | 1.63 |
| 11T | FePCYCl | 0.25 | $Cu(AcAc)_2$ | 0.05 | 20 | 25 | ~0 | 5.81 | 1.03 | 6.84 | 5.64 | 1.69 |
| 12T | $Fe(AcAc)_3$ | 0.50 | $Cu(AcAc)_2$ | 0.05 | 20 | 80 | 7.16 | 2.75 | 1.45 | 4.20 | 1.90 | 0.40 |
| 13T | Fe(II)PCY | 0.50 | - | - | 20 | 80 | ~0 | 3.56 | 6.05 | 9.61 | 0.59 | 0.15 |
| 14T | Fe(II)PCY | 0.50 | $Cu(AcAc)_2$ | 0.05 | 20 | 80 | ~0 | 3.37 | 4.80 | 8.17 | 0.70 | 0.20 |
| 15T | Fe(II)PCY | 0.50 | - | - | 20 | 25 | ~0 | 8.00 | 1.63 | 9.63 | 4.90 | 1.10 |
| 16T | Fe(II)PCY | 0.50 | $Cu(AcAc)_2$ | 0.05 | 20 | 25 | ~0 | 7.67 | 1.58 | 9.25 | 4.85 | 1.21 |
| 17T | Fe(II)PCY | 0.50 | Cu(II)PCY | 0.05 | 20 | 25 | ~0 | 8.76 | 2.06 | 10.82 | 4.25 | 0.86 |
| 18T | Fe(II)PCY | 0.50 | $Cu(OAc)_2$ | 0.05 | 20 | 25 | ~0 | 7.50 | 1.61 | 9.11 | 4.66 | 0.95 |
| 19T | Fe(II)PCY | 0.50 | Cu(II)PCY | 0.05 | 20 | 80 | ~0 | 3.81 | 5.31 | 9.12 | 0.72 | 0.18 |
| 20T | Fe(II)PCY | 0.50 | Cu(II)PCY | 0.05 | 20 | 25 | ~0 | 7.44 | 1.57 | 9.01 | 4.75 | 1.21 |
| 21T | Fe(II)PCY | 1.00 | - | - | 20 | 25 | ~0 | 6.70 | 2.42 | 9.12 | 2.76 | 1.02 |
| 22T | Fe(II)PCY | 0.50 | - | - | 20 | 80 | n/a | 0 | 0 | 0 | n/a | 8.93* |
| 23T | Fe(II)PCY | 0.25 | - | - | 20 | 25 | ~0 | 6.61 | 1.33 | 7.94 | 4.97 | 1.63 |
| 24T | Fe(II)PCY | 0.25 | - | - | 20 | 25 | ~0 | 5.38 | 1.08 | 6.46 | 4.98 | 1.95 |
| 25T | Fe(II)PCY | 0.04 | - | - | 20 | 25 | ~0.28 | 4.12 | 0.82 | 4.94 | 5.02 | 2.18 |

*15.0 g DTBP charged instead of TBHP.

With reference to Table 7, Examples 1T and 10T, which are run at the lower temperature of 20 °C and give ratios of wt.% ketone:wt.% alcohol of 4.96 and 5.21, respectively, may be compared with Example 4T, run at the higher temperature of 80 °C, which gives a ratio of 0.62, indicative of a greater alcohol proportion. Similarly, low temperature Examples 6T and 7T may be compared with hotter Example 5T; the ratios are 4.61 and 5.70 compared with 0.67. Further, low temperature Examples 15T and 16T may be compared with high temperature Example 13T; and low temperature Examples 17T and 20T may be compared with higher temperature Example 19T. In all cases, using a variety of catalyst systems, more alcohol is formed at the higher temperature, and more ketone is formed at the lower temperature.

It is also noted that di-tert-butyl peroxide (DTBP) is a useful by-product of this reaction. DTBP finds commercial use as a free radical initiator.

## EXAMPLES 40 TO 47

These Examples will illustrate that when TBHP is added to the stirred reaction mixture over an extended period using chloroferric phthalocyanine (FePCYCl) as the catalyst, a higher yield of alcohol plus ketone is obtained. There is only a small or negligible difference when ferrous phthalocyanine [Fe(II)PCY] is used as a catalyst, and long TBHP addition times are used. Procedure: a 250 ml round-bottom flask equipped with water-cooled condenser, thermometer and magnetic stirrer was charged with catalyst, n-dodecane, and tert-butyl alcohol (TBA). The flask was suspended in a water bath to which ice was added from time to time to maintain the temperature. Tert-butyl hydroperoxide (TBHP) was then added to the stirred reaction mixture by means of a syringe pump. At the end of the addition, the reaction was stirred for a further 5-10 hours at ambient temperature and then filtered. In this instance, the term "ambient

temperature" means 30° C ±5° C. Also, in each of of those Examples the amount of n-dodecane was 30.0 grams, the amount of TBA was 57.0 g, the amount of 90% TBHP was 33.0 g, and the amount of catalyst was 5.0 g. The products were determined by GC, and the data are presented in Table 8.

TABLE 8

| | | | ---------Products, wt.%--------- | | | |
|---|---|---|---|---|---|---|
| Ex. | Catalyst | Rate TBA Addn.(ml/hr) | Ketones | Alcohols | Total | TBHP (wt.%) Remaining |
| 40 | Fe(III)PCYCl | 0.458 | 5.548 | 2.623 | 8.171 | 2.376 |
| 41 | Fe(II)PCY | 0.458 | 3.229 | 5.652 | 8.881 | 3.434 |
| 42 | Fe(III)PCYCl | 2.292 | 3.851 | 0.690 | 4.541 | 2.371 |
| 43 | Fe(II)PCY | 2.292 | 4.032 | 3.381 | 7.413 | 0.789 |
| 44 | Fe(III)PCYCl | 4.584 | 2.546 | 0.756 | 3.302 | 1.272 |
| 45 | Fe(II)PCY | 4.584 | 5.220 | 3.226 | 8.446 | 0.114 |
| 46 | Fe(III)PCYCl | 11.46 | 3.673 | 0.688 | 4.361 | ~0 |
| 47 | Fe(II)PCY | 11.46 | 6.127 | 2.716 | 8.843 | 0.071 |

## EXAMPLES 48 TO 61

These are further Examples of the preparation of detergent range alcohols and ketones from n-dodecane and tert-butyl hydroperoxide using chloroferric phthalocyanine as the catalyst. It has been further discovered that when small amounts of an additive, such as iodosylbenzene are added to the reaction mixture, the rate of reaction is increased significantly. In this series of Examples, iodosylbenzene is the only additive employed and FePCYCl is the only catalyst used. The abbreviation "Inh. time" refers to the inhibition time, which is the time required for the reaction to proceed by at least 1%. The term $t_{\frac{1}{2}}$ refers to the half-life in minutes. The weight percentage of products was determined by GC. The results are reported in Table 9, and it is noted which of the following two procedures was used.

Procedure A: TBA and the catalyst were charged to a 250 ml, 3-necked round bottom flask equipped with a stirrer thermometer, water cooled condenser, and water bath. A tube led from the top of the condenser to a water filled gas-buret. TBHP was added all at once to the stirred mixture, the flask sealed, and the volume of gas (oxygen) given off at specified time intervals noted. A semilog plot of V-Voo versus time gave a pseudo first order rate constant ($10^3$ k $min^{-1}$ ). The products were determined by GC analysis of the filtered reactor effluent.

Procedure B: The same as the procedure used for the Examples of Table 6. The TBHP was added over a period of 4 to 6 hours.

TABLE 9

| | | | | | | Products in Effluent, Wt.% | | | | | | | | | |
|---|---|---|---|---|---|---|---|---|---|---|---|---|---|---|---|
| Ex. | C12 (g) | 90% TBHP (g) | TBA (g) | Cat. (g) | IB (g) | Acetone | TBA | C-12 | DTBP | Ketones | Alcohols | Inh. Time (min) | $10^3k$ $min^{-1}$ | $t_{\frac{1}{2}}$(min) | Procedure |
| 48 | 0.0 | 5.0 | 18.0 | 0.20 | 0.04 | 0.825 | 95.045 | 0.0 | 3.793 | 0.0 | 0.0 | 10 | 38.5 | 18 | A |
| 49 | 0.0 | 5.0 | 18.0 | 0.20 | 0.01 | 0.516 | 95.956 | 0.0 | 3.303 | 0.0 | 0.0 | 12 | 34.7 | 20 | A |
| 50 | 0.0 | 5.0 | 18.0 | 0.20 | None | 0.424 | 96.505 | 0.0 | 2.909 | 0.0 | 0.0 | 10 | 19.8 | 35 | A |
| 51 | 5.0 | 5.0 | 13.0 | 0.20 | None | 0.371 | 72.705 | 20.16 | 2.502 | 2.365 | 0.326 | 9 | 6.48 | 107 | A |
| 52 | 5.0 | 5.0 | 13.0 | 0.20 | 0.005 | 0.005 | 0.327 | 73.55 | 19.60 | 2.581 | 2.512 | 0.338 | 3.5 | 34.7 | A |
| 53 | 30.0 | 55.0 | 35.0 | 0.50 | 0.25 | 0.443 | 69.393 | 16.31 | 1.387 | 6.477 | 1.965 | - | - | - | A |
| 54 | 30.0 | 55.0 | 35.0 | 0.50 | 0.10 | 0.398 | 68.674 | 16.78 | 1.243 | 6.334 | 2.161 | - | - | - | B |
| 55 | 5.0 | 5.0 | 13.0 | 0.20 | 0.01 | 0.333 | 73.695 | 19.47 | 2.491 | 1.999 | 0.291 | 5 | 36.5 | 19 | A |
| 56 | 5.0 | 5.0 | 13.0 | 0.20 | 0.02 | 0.339 | 72.850 | 20.14 | 2.596 | 2.507 | 0.313 | 4 | 38.5 | 18 | A |
| 57 | 5.0 | 5.0 | 13.0 | 0.20 | 0.10 | 0.355 | 72.994 | 19.97 | 2.574 | 2.574 | 0.331 | 4 | 38.5 | 18 | A |
| 58 | 30.0 | 55.0 | 35.0 | 0.50 | 0.25 | 0.005 | 72.011 | 15.62 | 1.915 | 5.654 | 1.131 | - | - | - | B |
| 59 | 30.0 | 55.0 | 35.0 | 0.50 | 0.10 | 0.458 | 69.516 | 18.96 | 2.050 | 5.238 | 0.784 | - | - | - | B |
| 60 | 5.0 | 5.0 | 13.0 | 0.20 | 0.04 | 0.352 | 74.083 | 19.10 | 2.633 | 2.439 | 0.304 | 2 | 38.5 | 18 | A |
| 61 | 5.0 | 5.0 | 13.0 | 0.20 | None | 0.299 | 73.245 | 19.96 | 2.412 | 2.487 | 0.324 | 5 | 7.37 | 94 | A |

## EXAMPLES 62 TO 76

Examples 62 to 76 will illustrate the production of detergent range alcohol s and ketones from n-dodecane and tert-butyl hydroperoxide using a chloroferric phthalocyanine catalyst and sodium perchlorate as an additive. Sodium perchlorate is also used as an oxidant. However, in this case it might be better thought of as a ligand, but if so as at least a somewhat "reactive" ligand. It has been additionally discovered that the rate of reaction increases significantly when small amounts of sodium perchlorate are added to the reaction mixture. The abbreviations used in Table 9 are employed here as well. The term "R" refers to the correlation coefficient. The term "PYDiCr" refers to pyridinium dichromate. The procedure used was the same as that employed for Examples 48 to 61.

The reaction details and the product composition are set out below in Tables 10A and 10B respectively.

TABLE 10A

| Ex. | C-12(g) | TBA(g) | Cat.(g) | Additive | Additive (g) | Inh. time (min) | $10^3 k$ $min^{-1}$ | R | $t_{\frac{1}{2}}$ (min) |
|---|---|---|---|---|---|---|---|---|---|
| 62 | 0.0 | 18.0 | 0.20 | $NaClO_4$ | 0.10 | 4 | 59.9 | 0.978 | 11.7 |
| 63 | 0.0 | 18.0 | 0.20 | $NaBF_4$ | 0.10 | - | 18.4 | 0.960 | 37.6 |
| 64 | 0.0 | 18.0 | 0.20 | None | 0.0 | - | 13.6 | 0.962 | 51.0 |
| 65 | 0.0 | 18.0 | 0.50 | $NaBF_4$ | 0.10 | - | 34.8 | 0.965 | 19.9 |
| 66 | 0.0 | 18.0 | 0.20 | PYDiCr | 0.05 | - | 6.53 | 0.997 | 106 |
| 67 | 5.0 | 13.0 | 0.20 | None | 0.0 | - | 6.71 | 0.994 | 103 |
| 68 | 5.0 | 13.0 | 0.20 | $NaClO_4$ | 0.05 | - | 34.3 | 0.973 | 20.2 |
| 69 | 5.0 | 13.0 | 0.20 | $NaClO_4$ | 0.02 | - | 30.4 | 0.978 | 22.8 |
| 70 | 5.0 | 13.0 | 0.20 | $NaClO_4$ | 0.02 | 4 | 71.7 | 0.876 | 9.7 |
| 71 | 5.0 | 13.0 | 0.20 | $NaClO_4$ | 0.01 | <1 | 132 | 0.962 | 5.3 |
| 72 | 5.0 | 13.0 | 0.20 | $NaClO_4$ | 0.005 | 2 | 71.1 | 0.988 | 9.8 |
| 73 | 5.0 | 13.0 | 0.20 | $NaClO_4$ | 0.10 | 1 | 111 | 0.966 | 6.3 |
| 74 | 5.0 | 13.0 | 0.20 | $NaClO_4$ | 0.50 | <1 | 113 | 0.937 | 6.1 |
| 75 | 5.0 | 13.0 | 0.20 | None | 0.0 | 5 | 20.3 | 0.982 | 34.2 |
| 76 | 5.0 | 13.0 | 0.40 | None | 0.0 | 4 | 20.0 | 0.986 | 34.6 |

TABLE 10B

| | Products, Weight Percent, by GC | | | | | | |
|---|---|---|---|---|---|---|---|
| Ex. | Acetone | TBA | DTBP | TBHP | Ketones | Alcohols | K+A Total |
| 62 | 0.264 | 97.148 | 2.484 | 0.0 | - | - | - |
| 63 | 0.518 | 96.120 | 3.045 | 0.046 | - | - | - |
| 64 | 0.607 | 96.114 | 3.202 | 0.0 | - | - | - |
| 65 | 0.346 | 96.472 | 3.033 | 0.0 | - | - | - |
| 66 | 0.475 | 96.129 | 3.095 | 0.0 | - | - | - |
| 67 | 0.135 | 65.102 | 1.919 | 0.0 | 3.771 | 0.487 | 4.258 |
| 68 | 0.290 | 75.532 | 2.676 | 0.059 | 2.691 | 0.406 | 3.097 |
| 69 | 0.133 | 68.371 | 2.261 | 0.101 | 2.416 | 0.433 | 2.849 |
| 70 | 0.328 | 75.504 | 2.670 | 0.0 | 2.697 | 0.391 | 3.088 |
| 71 | 0.245 | 70.693 | 2.456 | 0.0 | 2.846 | 0.430 | 3.276 |
| 72 | 0.235 | 71.204 | 2.492 | 0.0 | 2.809 | 0.402 | 3.211 |
| 73 | 0.313 | 72.734 | 2.701 | 0.0 | 2.556 | 0.376 | 2.932 |
| 74 | 0.254 | 70.911 | 2.562 | 0.0 | 2.888 | 0.416 | 3.304 |
| 75 | 0.200 | 67.230 | 2.227 | 0.0 | 3.375 | 0.790 | 4.165 |
| 76 | 0.153 | 53.969 | 1.832 | 0.0 | 4.778 | 1.200 | 5.978 |

## EXAMPLES 77 TO 82

These Examples will demonstrate that potassium superoxide is a useful additive when used with the chloroferric phthalocyanine catalyst of the present invention. It is expected that rubidium and cesium superoxides would also be useful in this respect. High yields of the ketone/alcohol mixture may be obtained. The procedure was as follows: A 250 ml flask was charged with catalyst(s), n-dodecane (30.0 g), tert-butyl alcohol (35.0 g), and this mixture was vigorously stirred. In Examples 77 to 82, 0.50 g of Fe(III)PCYCl catalyst was used throughout. A mixture of 33.0 g 90% tert-butyl hydroperoxide and 22.0 g of tert-butyl alcohol was added slowly to this stirred mixture over several hours. The reactions at 25° C were controlled by means of a water bath. The reactions at 80° C were controlled by means of a heating mantle. The reaction mixtures were stirred for a total of 20 nours, and filtered through fluted filter paper. The results shown in Table 11 below were obtained by GPC.

Again, although potassium superoxide is often an oxidant. it is suspected that it may be acting as a ligand in this case. If it is acting as a ligand, it is not acting in the same way as imidazole or lithium borate. It may be functioning as a kind of reactive ligand, but this is not known for certain.

TABLE 11

| | | | | ----------Products, Wt. Percent by GPC---------- | | | | | |
|---|---|---|---|---|---|---|---|---|---|
| Ex. | Additive | Additive (g) | Temp (°C) | TBHP | Ketone | Alcohol | Total | Ketone/Alcohol | DTBP. |
| 77 | $KO_2$ | 0.10 | 25 | ~0 | 6.01 | 1.12 | 7.13 | 5.36 | 1.81 |
| 78 | $KO_2$ | 0.30 | 25 | ~0 | 6.52 | 1.19 | 7.71 | 5.45 | 1.68 |
| 79 | $KO_2$ | 0.50 | 25 | ~0 | 6.44 | 1.19 | 7.63 | 5.41 | 1.71 |
| 80 | $KO_2$ | 0.30 | 80 | ~0 | 2.94 | 5.49 | 8.43 | 0.54 | 0.18 |
| 81 | - | - | 25 | 0.05 | 4.26 | 0.92 | 5.19 | 4.63 | 1.96 |
| 82 | $K_2S_2O_8$ | 0.50 | 25 | 0.11 | 5.14 | 0.90 | 6.04 | 5.71 | 1.77 |

## EXAMPLES 83 TO 90

These Examples will illustrate the use of tetrabutyl ammonium bromide (TBAB) in conjunction with an additive, such as potassium superoxide ($KO_2$) from the previous Examples 577 to 82. In the presence of potassium superoxide and TBAB as well as the chloroferric phthalocyanine catalyst, an increased yield of detergent range alcohols and ketones is obtained.

In the procedure of Examples 83 to 90, a 250 ml flask was charged with catalyst(s), n-dodecane (30.0 g), and tert-butyl alcohol (35.0 g), and this mixture was vigorously stirred. A mixture of 33.0 g 90% tert-butyl hydroperoxide and 22.0 g of tert-butyl alcohol was added slowly to this stirred mixture over several hours. The reactions were all conducted at 25° C, and temperature was controlled by means of a water bath. The reaction mixtures were stirred for a total of 20 hours and filtered through fluted filter paper. The results shown in Table 12 below were obtained by GPC. Example 62 which uses no additive is shown for comparison.

TABLE 12

| | | | | | ----------Products, Wt. % by GPC---------- | | | | | |
|---|---|---|---|---|---|---|---|---|---|---|
| Ex. | Catalyst | (g) | Additive | Additive (g) | TBHP | Ketone | Alcohol | Total | Ketone/Alcohol | DTBP |
| 83 | Fe(III)PCYCl | 0.50 | CTMAC | 0.25 | 0.05 | 4.46 | 0.70 | 5.16 | 6.37 | 2.07 |
| 84 | Fe(III)PCYCl | 0.50 | TBAB | 0.25 | 0 | 6.00 | 1.09 | 7.09 | 5.50 | 1.57 |
| 85 | Fe(III)PCYCl | 0.50 | THAB | 0.25 | 0.25 | 4.89 | 0.85 | 5.74 | 5.75 | 1.85 |
| 86 | Fe(III)PCYCl | 0.50 | MBTPPC | 0.25 | 0.17 | 4.05 | 0.70 | 4.75 | 5.78 | 2.18 |
| 87 | Fe(III)PCYCl | 0.50 | $KO_2$<br>CTMAC | 0.50<br>0.25 | 0.05 | 7.26 | 0.95 | 8.21 | 7.64 | 1.28 |
| 88 | Fe(III)PCYCl | 0.50 | $KO_2$<br>TBAB | 0.50<br>0.25 | 0 | 7.03 | 1.41 | 8.44 | 4.99 | 1.43 |
| 89 | Fe(III)PCYCl | 0.50 | $KO_2$<br>THAB | 0.50<br>0.25 | 0.07 | 6.93 | 1.31 | 8.24 | 5.29 | 1.36 |
| 81* | Fe(III)PCYCl | 0.50 | - | - | 0.05 | 4.26 | 0.92 | 5.18 | 4.63 | 1.96 |
| 90 | $KO_2$ | 2.00 | THAB | 0.25 | 14.17 | 0 | 0 | 0 | - | 0.06 |

*Provided from Table 11 for ease of comparison.

| GLOSSARY | |
|---|---|
| CHP | Cumene hydroperoxide |
| $Cr(AcAc)_3$ | Chromium (III) acetylacetonate |
| $Cu(OAc)_2$ | Copper (II) acetate |
| DTBP | Di-tert-butyl peroxide |
| $Fe(AcAc)_3$ | Iron or Ferric (III) acetylacetonate |
| IM | Imidazole |
| $Ru(AcAc)_3$ | Ruthenium (III) acetylacetonate |
| TBA | tert-butyl alcohol |
| TBHP | tert-butyl hydroperoxide |
| Fe(II)DCPA | Dicyano bis-(1,10)-phenanthrolene iron(II) |
| $Fe_2[Col.]_3[Cit.]_3$ | Iron choline citrate |
| Fe(III)DETAPA | Diethylenetriamine pentaacetic acid iron (III), disodium salt |
| CTMAC | Cetyl trimethylammonium chloride |
| $Cu(AcAc)_2$ | Copper (II) acetylacetonate |
| CuPCY | Cuprous phthalocyanine |
| Cu(II)PCY | Copper (II) phthalocyanine |
| Fe(II)PCY | Ferrous phthalocyanine |
| FePCYCl | Chloroferric phthalocyanine, also denoted as Fe(III)PCYCl |
| IB | Iodosylbenzene |
| MBTPCC | Methylbenzyl triphenyl phosphonium chloride |
| PYDiCr | Pyridinium dichromate |
| TBAB | Tetrabutyl ammonium bromide |
| THAB | Tetrahexyl ammonium bromide |

## Claims

1. A process for the production of a mixture of detergent range alcohols and ketones by reacting an alkane having 10 to 18 carbon atoms with a hydroperoxide in the presence of a catalyst, characterized in that the catalyst is a transition metal compound selected from:

(a) dicyano bis-(1,10)-phenanthrolene iron (II),
(b) iron, ruthenium or chromium acetylacetonate, and
(c) an iron, manganese or cobalt phthalocyanine or metallo-porphine.

2. A process according to Claim 1 characterized in that the hydroperoxide is cumene hydroperoxide, tertiary butyl hydroperoxide or a mixture thereof.

3. A process according to Claim 1 or 2 characterized in that the catalyst is iron acetylacetonate, with copper acetate as a co-catalyst.

4. A process according to Claim 1 characterized in that the catalyst is ferrous phthalocyanine, chloroferric phthalocyanine, iron (II) meso-tetraphenyl porphine chloride, manganese (II) tetraphenyl porphine acetate, cobalt (II) meso-tetraphenyl porphine or a mixture thereof.

5. A process according to Claim 4 characterized in that the catalyst is employed in the additional presence of a ligand.

6. A process according to Claim 5 characterized in that the ligand is an imidazole, lithium borate or a mixture thereof.

7. A process according to Claim 4 characterized in that the catalyst is chloroferric phthalocyanine in the additional presence of an additive selected from alkali metal superoxides, alkali metal perchlorates, iodosylbenzene and mixtures thereof.

8. A process according to Claim 7 characterized in that the additive is an alkali metal superoxide and tetrabutyl ammonium bromide is present.

9. A process according to any one of Claims 4 to 8 characterized in that the hydroperoxide is added continuously over a period of at least 2 hours.

10. A process according to any one of Claims 4 to 9 characterized in that the temperature is adjusted to control the nature of the product, being raised to increase the proportion of alcohols in the product, and lowered to increase the proportion of ketone in the product.